# EUROPEAN PATENT APPLICATION

(11) **EP 2 893 903 A1**
(43) Date of publication of application: **15.07.2015**
(21) Application number: 13835731.4
(22) Date of filing: 06.09.2013
(51) Int. Cl.: A61F 2/14

(54) **IMPLANTABLE DEVICE FOR MOULDING THE CURVATURE OF THE CORNEA**

(30) Priority: 07.09.2012 US 201261698177 P; 05.09.2013 US 201314018866
(71) Applicant: Mediphacos Indústrias Médicas S/A, CEP:30575-815 - Belo Horizonte - MG (BR)
(72) Inventor: SOARES, Marcelo Francisco Pessoa, CEP: 30575-815 Belo Horizonte - MG (BR); CAMARGOS, Marcelo Duarte, CEP: 30220-260 Belo Horizonte - MG (BR); COSKUNSEVEN, Efekan, CEP: 34337 Etiler- Istanbul (TR)
(74) Representative: Soldatini, Andrea
(86) International application number: PCT/BR2013/000345
(87) International publication number: WO 2014/036622

(57) **Abstract**

The present invention relates to anintrastromal cornealdevice for reshaping the curvature of the cornea comprising a substantially circular structure having a convex surface provided with openings for biological exchange between the layers of the cornea, said substantially circular structure comprising the shape of a circular segment or of a spherical /aspheric cap provided or not with a central opening. Said device further comprises means for promoting thereshaping of the cornea, so that it assumes a curvature closer to the intended curvature, thus providing a significant reduction of visual disturbances caused by corneal ectatic conditions such as keratoconus.

## Description

### Field of the Invention

The present invention belongs to the field of ophthalmic implants and it relates to a device comprised basically of a structure which reshapes the curvature of the cornea thereby correcting the morphologic deformations and ocular refractive errors associated with corneal ectatic diseases.

### Background of the Invention

As it is known in the art, the intrastromalimplant uses small devices, which are comprised of rings or ring segments that are commonly produced in polymethyl methacrylate, which are inserted between the layers of the cornealstroma on each side of the pupil (that is, off visual axis), through a small incision made on the periphery of the cornea.Such devices were originally developed for the treatment and correction of myopia;however such procedures are rare so that the main application of such devices has been mainly limited to treatment of keratoconus- a non-inflammatory degenerative eye disease, which causes structural changes in the cornea that ultimately leave it thinner and having a more conical shaped surface (ectasia) than its conventional gradual curvature does, thereby causing a lossto the quality of vision.

The symptoms resulting fromkeratoconus include distortion of images, generation of multiple images, photophobia,myopia and irregular astigmatism. In the current art,one or two ring segments are implanted within the cornealstroma in order to flatten the corneal curvature and, thereby,reduce the recited visual effects.Among the products that are already known for asintrastromal implants, it may be recited the following ones:
- Intacs (Addition Technology Inc.):comprises ring segments having thicknesses between 0.25 mm and 0.45 mm and arc of 150°.It also comprises a variable radius of between 2.5 mm and 3.5 mm, hexagonal cross section, 6.77 mm inner diameter and 8.10 mm outer diameter;
- Keraring (Mediphacos):ring segments of thickness between 0.15 mm and 0.35 mm, arcs of 90°, 120°, 160° and 210°.It has variable radius of 2.5 mm, 2.75 mm or 3mm;
- Ferrara Ring:produced of yellow PMMA, diameters of 5 and 6 mm, straight-based triangular cross shape, arcs ranging between 90 and 210° and a thickness ranging from 0.15 to 0.30 mm.4.4 mm inner radius and 5.6 mm outer radius;
- KERATACx:thicknesses ranging from 0.1 to 0.35 mm in steps of 0.025 mm.

Several published documents address the issue of using intrastromal rings for the treatment of keratoconus, such as, for example:
- TORQUETTI, L.; FERRARA, P..Intrastromal rings still useful in Keratonocus. EyeWorld, US, 2010:88-90.
- ERTAN, A..Intracorneal rings for keratoconusanskeratectasia. JournalofCataractandRefractiveSurgery, 2007(33): 1303-1314.
- ALIÓ, L. L. et al. Intracorneal rings to correct corneal ectasia alter laser in situ keratomileusis. JournalofCataractandRefractiveSurgery, 2002(28): 1568-1574.

Several patent documents also describe said rings. US Patent 6,966,972 discloses a hybrid structured ring being formed by a central nucleus of rigid material coated with a more flexible material and, analogously,US Patent 6,214,044 also emphasizes the constructive composition of the rings comprising high and low elastic modulus materials, while document US Patent 5,888,243 discloses rings having hollow inner region.

US 8,394,140 refers to implants composed of rings which may have a circular or substantially elliptical cross-section shape, nevertheless they merely provide for a ring shapethat is not capable of broadly adjusting the full curvature of the cornea, thereby presenting limited results.

US Patent 5,653,752 and US Patent 5,693,092, in turn, describe rings that can be superimposed in order to provide an increase in the final implant thickness.

However, the devices currently known in the art act by the principle of corneal remodeling described by Jose IgnacioBarraquer which has been known in ophthalmologic medicine as "Barraquer Thickness Law." According to Barraquer Law, addition of tissue to the periphery of the cornea causes a central flattening of its curvature. In this sense, implants of intracorneal rings act as the "tissue" added to the corneal periphery, thus producing central flattening that may reduce visual symptoms associated with keratoconus. However, all of the intracorneal implants present low predictability, that is, it is not possible to precisely estimate the final curvature of the cornea. Such a low predictability may generate undesirable effects such as refractive undercorrectionor overcorrection, induction of astigmatism or high order optical aberrations that compromise the quality of vision.Hence, keratoconus is exclusively treated by flateningthe steep corneal curvaturethat it causes without, however, inducing the cornea to attain a predetermined curvature desired to maximize the quality of vision of each case.

Additionally, the existing intracorneal implants cover a substantially limited corneal area, having a reduced width, usually less than 1 mm such that it does not comprise nutrient diffusion to corneal stroma.

Thus, one may note that the current state of the art lacks solutions for improving the treatment of corneal ectasiasby utilizing means for inducing the surface of the cornea to obtain a predetermined curvature which can provide for a better quality od vision.

### Objects of the Invention

Therefore, in view of the above, it is one object of the present invention to provide an intrastromal corneal device for the reshaping the curvature of the cornea, capable ofreshaping the curvature of the cornea according to its own curvature.

In addition, it is an object of the present invention to provide an intrastromal device that comprises cross-sections with variable curvature radii to reshape the curvature of the cornea without compromising the exchange of gases and nutrients in corneal tissue.

### Summary of the Invention

The present invention achieves the above-mentioned objects by means of an intrastromalcorneal device forreshapingof curvature of the cornea, said device being intended to besurgically implanted within human corneas, between its layers, and designed to alter the curvature of the cornea for the treatment of corneal ectasias.

In the preferred embodiment of the invention, said intrastromal corneal device for reshaping of corneal curvature comprises a substantially circular structure with convex surface and cross-section having a variable curvature radius proportional to the intended curvature radius to be achieved on the treated cornea, wherein said convex surface comprises at least an opening for biological exchange between the corneal layers, and wherein said substantially circular structure has the shape of a circular segment or of a spherical cap being provided or not with at least one opening on its surface.

The objects of the invention are also accomplished by means of an intrastromalcorneal device comprising a material with an elastic modulus greater that the elastic modulus of the human cornea, said modulus being preferable between 0.6 and 8.500 Mpa.

Thus, preferably, said intrastromal device will be made of methyl methacrylate polymers or copolymers or other biocompatible polymers, or optionally made of biocompatible metal materials such as platinum, titanium, etc.

To this effect, in a preferred embodiment, said intrastromal corneal device of the present invention comprise at least a circular segment having convex surface provided with openings, wherein said circular segment has in its cross-section a curvature radius ranging from 4.5 mm to 17.0 mm.

Alternatively, the device of the present invention comprises at least a circular body having convex surface provided with at least one central opening, wherein said convex surface further comprises cavities disposed along its surface to allow for stromal nutrition.

In a further embodiment, said device comprises at least two circular segments, each defined by convex surfaces having inner cavities, wherein said circular segments are linked to at least a central ring by means of rods.

Accordingly, the invention comprises means for promoting remodeling of the cornea so that same acquires a predetermined curvature closer to the ideal curvature for that patient, thus providing a significant reduction in the negative visual effects caused by corneal ectasias. Furthermore, it comprises means to alter curvatures of the cornea according to the curvature of the device.

### Brief Description of the Drawings

The present invention will be detailed in figures below, in which:
Figures 1.1, 1.2, 1.3, 1.4 and 1.5 illustrate a preferred embodiment of the intrastromal device in the shape of a capsegment having a reduced arc length, in a perspective view, top view, front view, side view and enlarged detail view, respectively;
Figures 2.1, 2.2, 2.3 and 2.4 illustrate a second embodiment of the intrastromal device according to the present invention, in the form of capsegment having a greater arc length, in a perspective, top view, front view and enlarged detail view, respectively;
Figures 3.1, 3.2, 3.3 and 3.4 illustrate a third embodiment of the intrastromalaccording to the present invention, in the form of aspheric or spherical cap having a central opening, respectively in perspective view and top view, front view and enlarged detail view;
Figures 4.1, 4.2, 4.3 and 4.4 illustrate another embodiment of the intrastromal device according to the present invention in the form of perimetral cap without having a central opening, in perspective view, top view, front view and side view, respectively;
Figures 5.1, 5.2, 5.3 and 5.4 illustrate a further embodiment of the intrastromal device in the form of circular segments linked to a central ring in perspective, top, frontal e side views.

### Detailed Description of the Invention

According to the schematic figures mentioned herein above, a few examples of possible embodiments of the present invention will be described below in more details, by way of example only and without limitation, since the object of the present invention may comprise different details and structural and dimensional aspects without thereby departing from the scope of the desired protection.

Thus, the present invention relates to an intrastromal corneal device for reshaping the curvature of the cornea comprising an open body or cap-shaped having a curvature at its base.Therefore, the device comprises a structure mainly intended toreshape the curvature of the cornea according to its own curvature, thereby correcting visual disturbances that accompany corneal ectasias, differentiating it from existing ones as it has a more elongated and curved profile, besides having hollow spaces along its body in order to allow gas and nutrient exchange throughout the cornea.

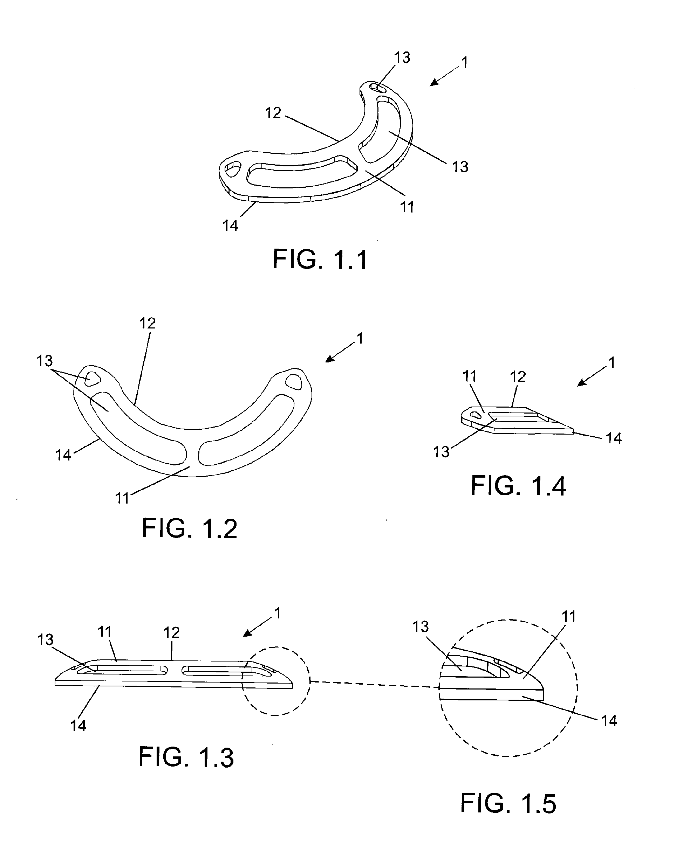

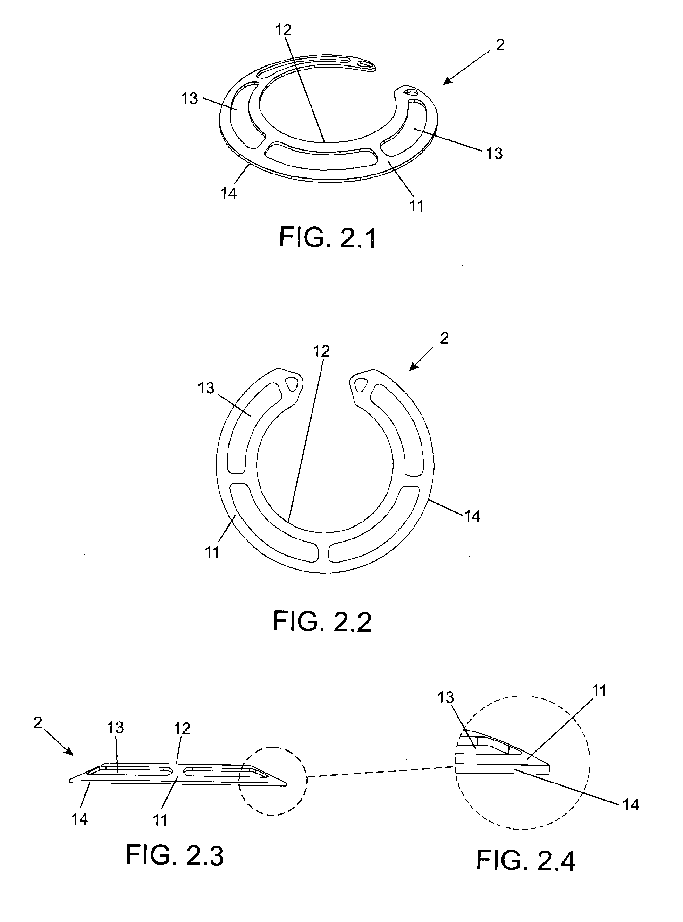

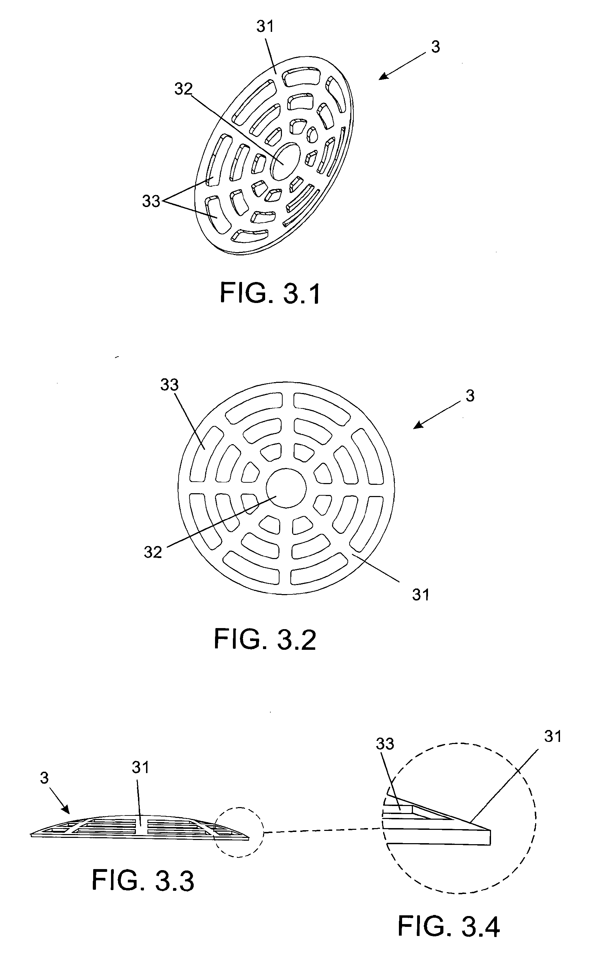

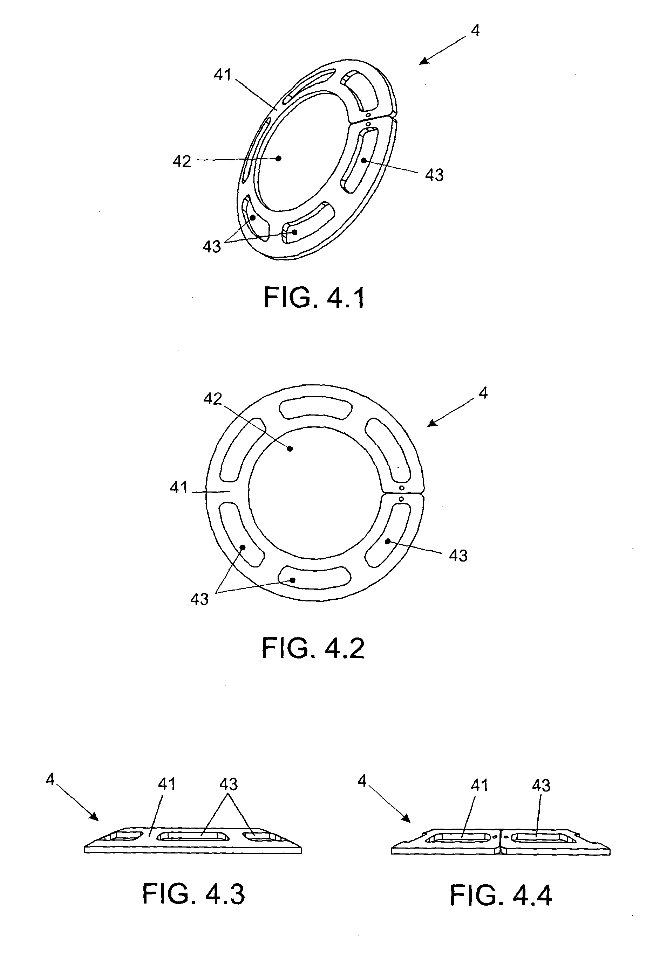

The device according to the present invention can be produced in two basic shapes:the first one, illustrated in Figures 1 and 2, basically comprises an open circular structure in the shape of an arc segment;and the second shape, illustrated in Figures 3 and 4, comprises a structure in the form of a substantially aspheric or spherical cap.

In Figures 1.1 to 1.5, it is illustrated a first embodiment of said intrastromal device, which is basically defined by a circular segment 1 having a smoothly convex surface 11 having central gaps 13.It should be observed that the length of the arcs may vary, wherein the manufacture and commercialization of different models having arcs of different lengths can be carried out.

In those figures, it is observed an elevation / angle in its inner edge 12, which is due to its curvature - a differential that is responsible for reshaping the cornea into a predetermined and desired form.Figures 1.2 and 1.5 show the device 1 in top view and a detail of the side view profile clearly depicting the elevated position of the inner edge 12 with a curved profile between the outeredge 14 and the inner edge 12. From the top view shown in Figure 1.2, it can be seen the presence of central gaps 13 along its structure. Said gaps 13 are crucial to ensure the biocompatibility of the device as they allow the cornea to continue with its processes of transferring nutrients and oxygen between the inner and outer layers thereof.

Figures 2.1 to 2.4 illustrate a second embodiment showing an implant having greater arc length, wherein an indication of the length suitable for each patient is made by the physician.This configuration havingan obtuse arc 2 is also defined by a circular segment with a smoothly convex surface 11 having central gaps 13 for transference / passage of biological material.

Said implant may also be in the form of a spherical or aspheric cap, as shown in Figures 3 and 4. The aspheric shape is intended to correct a spherical aberration of the patient's eye, aiming at increasing visual acuity thereof.This embodiment is completely novel compared to the devices already known from the state of the art.

In the embodiment illustrated in Figures 3.1 to 3.4, there is a circular body 3 with a convex surface 31 provided with a central opening 32 and cavities 33 disposed along its surface 31.

Further, in the embodiment in the form of a perimetral cap illustrated in Figures 4.1 to 4.4, the circular body 4also having a convex surface 41 is provided with a broad central opening, but still contains cavities 42 for biological exchanges.

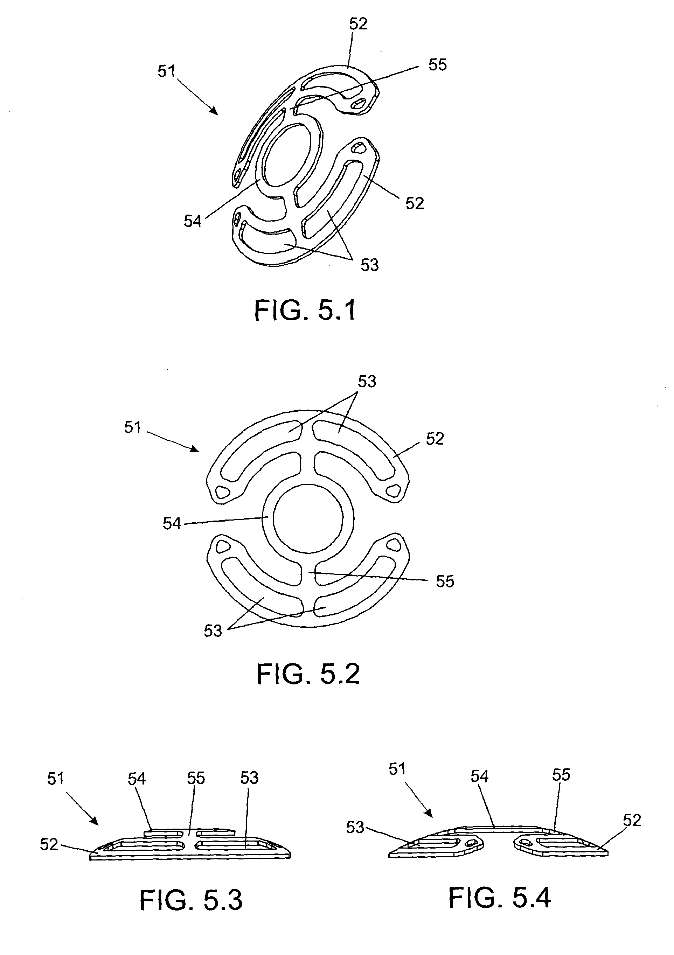

The alternative embodiment illustrated in Figure 5 is basically defined by two circular segments 51, each being defined by convex surfaces 52 with inner cavities 53, wherein said circular segments 51 are linked to another by means of a central ring 54 which is interconnected to same by means of rods 55.

From the embodiments of devices for intrastromalimplants according to the present invention, it is possible to promote the reshaping of the curvature of the cornea according to the implant curvature and having a profile thatis almost spherical, in a shape very similar to that of a healthy eye and having a conventional corneal topography. Furthermore, since said device has a more elongated profile, particularly that one shown in Figures 2.1 to 2.4, it also contributes to the patient's cornea to deform according to the shape of the implant.

Another important advantage of the device of the present invention over the similar known devices relates to cavities 13, 33 and 42 that allow for nutrient and gas exchange throughout the cornea to occur, thereby contributing to maintaincorneal health.

It should be clarified that a conventional ring adds volume to the periphery of the cornea, causing it to become flatter.The proposal herein presented contains differences that maximize the benefits thereof. The greatest differential is that the cross section thereof has a previously defined curvature, as it can be seen in the profile shown in Figures 1.5, 2.4, 3.4 and 4.4. The convex surface and largest width of the implant over the conventional ones cause the cornea to deform according to the shape of the device.Thus, the cornea assumesanew radius of curvature, thereby allowing for animproved quality of vision for patients who suffer from corneal ectasias.The greatest width of the implant or even the possibility of constructing the cap are only possible because the embodiments comprise openings along their bodies, and saidopeningsallow for a continuous exchange of gases and nutrients between the inner and outer layers of the cornea, which significantly contributes to the biocompatibility thereof.

In addition, in order to ensure that the cornea is adapted to the device shape, this is made of a material with constant elastic modulus that allows it to be more rigid than the cornea, so that the opposite effect does not happen, that is, the implant will deforming according to an inappropriate curvature of the cornea.

Since the elastic modulus of the cornea is approximately 0.29 ± 0.06 Mpa is recommended to use materials having elastic modulus between 0.6 and 8.500 Mpa-such as, for example, methyl methacrylate polymers and copolymer (for the sake of information, it is worthy to mention that the elastic modulus of acrylic is 3.000 Mpa and Nylon 6/10 has a elastic modulus of 8.300 Mpa.

It should be clarified that, preferably, the implants should be made of materials that are transparent to visible light.

It is important to emphasize that the foregoing description has only been presented for purposes of illustration of the preferred embodiments of the claimed device. Therefore, as it is well known to those skilled in the art, numerous constructive modifications, variations and combinations of the elements exerting substantially the same function to obtain the same results are also herein contemplated and are within the scope of protection defined by the appended claims.

## Claims

1. Intrastromalcorneal device for reshaping the curvature of the cornea, wherein said device is intended to be surgically implanted within human corneas, between their layers, and it is designed to reshape the curvature of the cornea for the treatment of corneal ectasias, **CHARACTERIZED in that** the device comprises a substantially circular structure having a convex surface, wherein:
- said convex surface comprises at least one opening for biological exchange between the corneal layers; and
- said substantially circular structure comprises the shape of a circular segment or of a spherical or aspherical form having a circumscribed diameter ranging from 2.0 mm to 13.0 mm;
the cross-section of said device having a variable curvature radius ranging from 4.5 mm to 17.0 mm.

2. Intrastromalcorneal device,in accordance with claim 1, **CHARACTERIZED in that** said substantially circular structure comprises at least one opening on its surface.

3. Intrastromal device, in accordance with claim 1, **CHARACTERIZED in that** it comprises a material with an elastic modulus greater than the elastic modulus of a human cornea.

4. Intrastromal corneal device, in accordance with claims 1 and 3, **CHARACTERIZED in that** it comprises a material with an elastic modulus ranging from 0.6 to 8.500 Mpa.

5. Intrastromal corneal device, in accordance with claims 1 and 3, **CHARACTERIZED in that** it can be made of methyl methacrylate polymers or copolymers or other biocompatible polymers.

6. Intrastromal corneal device, in accordance with claims 1 and 3, **CHARACTERIZED in that** it is made of biocompatible metal materials such as platinum, titanium, etc.

7. Intrastromal device, in accordance with claim 1, **CHARACTERIZED in that** it comprises at least one circular segment 1 having convex surface 11 provided with central openings 13, wherein said circular segment 1 contains a inner edge 12 in a more elevated position than the position of outer edge 14 to generate the intended curvature radius.

8. Intrastromal device, in accordance with claim 1, **CHARACTERIZED in that** it comprises at least a circular body (3, 4) having a convex surface (31, 41) provided with at least a central opening (31, 41), said convex surface (31, 41) further comprising openings (33, 43) disposed along the surface thereof.

9. Intrastromal corneal device, in accordance with claim 1, **CHARACTERIZED in that** it comprises at least two circular segments (51), each being defined by convex surfaces (52) with inner openings (53), wherein said circular segments (52) are linked to at least a central ring (54) by means of rods (55).

10. Intrastromal device, in accordance with claim 1, **CHARACTERIZED in that** it comprises means for promoting cornea remodeling so that the cornea assumes a curvature closer to the desired one, thereby significantly reducing the vision disturbing effects caused by corneal ectasias.

11. Intrastromal corneal device, in accordance with claim 1, **CHARACTERIZED in that** it comprises means for altering the curvature of the cornea in conformity with the device curvature.

## Amended claims

### Amended claims under Art. 19.1 PCT

**1.** Intrastromal corneal device for reshaping the curvature of the cornea, wherein said device is intended to be surgically implanted within human corneas, between their layers, and it is designed to reshape the curvature of the cornea for the treatment of corneal ectasias, **CHARACTERIZED in that** the device comprises a circular structure having a convex surface, wherein:
- said convex surface comprises at least one opening for biological exchange between the corneal layers; and
- said circular structure comprises the shape of a circular segment or of a spherical or aspherical form having a circumscribed diameter ranging from 2.0 mm to 13.0 mm;
the cross-section of said device having a variable curvature radius ranging from 4.5 mm to 17.0 mm.

**2.** Intrastromal corneal device, in accordance with claim 1, **CHARACTERIZED in that** said substantially circular structure comprises at least one opening on its surface.

**3.** Intrastromal device, in accordance with claim 1, **CHARACTERIZED in that** it comprises a material with an elastic modulus greater than the elastic modulus of a human cornea.

**4.** Intrastromal corneal device, in accordance with claims 1 and 3, **CHARACTERIZED in that** it comprises a material with an elastic modulus ranging from 0.6 to 8.500 megapascals.

**5.** Intrastromal corneal device, in accordance with claims 1 and 3, **CHARACTERIZED in that** it can be made of methyl methacrylate polymers or copolymers.

**6.** Intrastromal corneal device, in accordance with claims 1 and 3, **CHARACTERIZED in that** it is made of biocompatible metal materials such as platinum, titanium.

**7.** Intrastromal device, in accordance with claim 1, **CHARACTERIZED in that** it comprises at least one circular segment 1 having convex surface 11 provided with central openings 13, wherein said circular segment 1 contains a inner edge 12 in a more elevated position than the position of outer edge 14.

**8.** Intrastromal device, in accordance with claim 1, **CHARACTERIZED in that** it comprises at least a circular body (3, 4) having a convex surface (31, 41) provided with at least a central opening (31, 41), said convex surface (31, 41) further comprising openings (33, 43) disposed along the surface thereof.

**9.** Intrastromal corneal device, in accordance with claim 1, **CHARACTERIZED in that** it comprises at least two circular segments (51), each being defined by convex surfaces (52) with inner openings (53), wherein said circular segments (52) are linked to at least a central ring (54) by means of rods (55).

**10.** Intrastromal device, in accordance with claim 1, **CHARACTERIZED in that** it comprises means for promoting cornea remodeling so that the cornea assumes a curvature closer to the desired one, thereby significantly reducing the vision disturbing effects caused by corneal ectasias.

**11.** Intrastromal corneal device, in accordance with claim 1, **CHARACTERIZED in that** it comprises means for altering the curvature of the cornea in conformity with the device curvature.
